# EUROPEAN PATENT SPECIFICATION

(11) **EP 1 247 541 B1**
(45) Date of publication and mention of the grant of the patent: **23.08.2006**
(21) Application number: 01303181.0
(22) Date of filing: 03.04.2001
(51) Int. Cl.: A61M 25/06, A61B 17/34

(54) **Needle with guide means**
Nadel mit Führungsvorrichtung
Aiguille avec moyens de guidage

(43) Date of publication of application: 09.10.2002
(73) Proprietor: Unomedical Limited, Redditch, Worcestershire B98 9N1 (GB)
(72) Inventor: Radhakrishna, Subrahmanyam, Dr., Solihull, Birmingham B90 1SJ (GB)
(74) Representative: Nielsen, Henrik Sten

(56) References cited:
- FR-A- 2 292 457
- GB-A- 2 343 845
- US-A- 5 375 588
- US-A- 5 954 670
- US-A- 5 964 670

## Description

This invention relates to a needle that can be used to perform medical procedures, such as Internal jugular cannulation much more safely than by the methods presently available.

A small-bore shorter needle is often used to seek out the primary location of a structure before passing a longer wide bore needle. This is to prevent accidental damage to the vital structures in the vicinity. But the smaller needle has nothing to guide the larger needle by to the identified site. The only guide for the larger needle is the direction of the smaller needle. This frequently results in loss of direction, and delay or failure of the procedure.

The object of the present invention is to provide a needle that can precisely guide a longer wide bore needle to the identified site. Accordingly, this invention provides the smaller needle with an attachment to guide the larger needle to its target. This attachment must be made of tough, unbreakable material that can be sterilised for use during medical procedures.

A preferred embodiment of the invention will now be described with reference to the accompanying drawing in which:
Figure 1 shows side view of the invention
Figure 2 shows the invention as viewed from the hub end of the needle
Figure 3 shows the sniper needle with a large bore needle passed through the attachment.

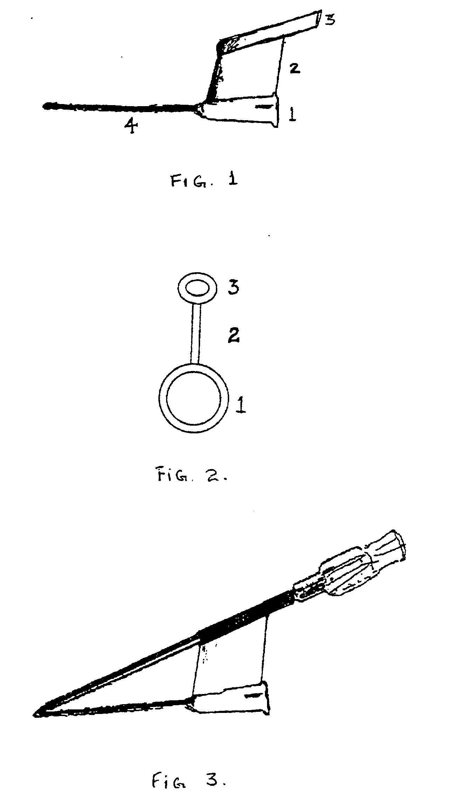

As shown in Figure 1, the sniper needle is characterised by the hub (1), which is co-axial to the needle (4), and to, which is attached a collateral web (2). A long narrow tubular passage (3) is attached to the upper end of the web and runs the whole length of its upper border. Figure 2 shows the arrangement as seen from the hub of the needle.

The tubular passage slopes from behind forwards towards the tip of the needle so that any other needle passed through this would come to lie with its sharp point approximated close to the sharp point of the sniper needle as shown in figure 3. The length, the angle and the diameter of the tubular passage are crucial to the construction and can be varied depending on the nature and the site of the procedure. When the whole length of the large bore needle used for a particular procedure is inserted through the tubular passage as shown in Figure 3, the proximal part of the passage would be against the hub of the larger needle. At the same time, the tips of the two needles would be close together. The larger needle should not project beyond the tip of the sniper needle. The diameter of the tubular passage should be just enough for the large bore needle to pass through and should not allow side to side swaying of the large needle along its long axis. To perform a procedure such as internal jugular cannulation the sniper needle is first attached to a syringe. The needle is inserted through the skin with a constant suction applied through the syringe. The site would be identified by the flow back of body fluids into the syringe. Once the site is reached, the needle is held by the non-dominant hand to maintain the position and the appropriate large needle is passed to its full length through the tubular passage to hit the target. Once the procedure is completed, the needles can be withdrawn simultaneously.

## Claims

1. A needle (4) comprising guide means is **characterised by** a hub (1) located coaxial to the needle (4) and from this hub projects a collateral web (2) with a tubular passage (3) designed to precisely guide a large bore needle to the tip of the needle with guide means (4) said tubular passage being orientated so that the axis of the largebore needle intersects the axis of the needle with guide means (4), thereby allowing safe and accurate insertion of the large bore needle into blood vessels like the internal jugular vein.

## Patentansprüche

1. Nadel (4) umfassend eine Führungsvorrichtung ist **durch** eine koaxial zur Nadel (4) angeordnete Hülse (1) **gekennzeichnet**, und von dieser Hülse ragt eine seitliche Bahn (2) mit einem röhrenförmigen Durchgang (3) hervor, der derart gestaltet ist, dass er eine große Bohrkanüle zur Spitze der Nadel mit Führungsvorrichtung (4) genau führt, wobei der röhrenförmige Durchgang (3) derart orientiert ist, dass die Achse der großem Bohrkanüle die Achse der Nadel mit Führungsvorrichtung (4) durchschneidet, wodurch eine sichere und präzise Einführung der großen Bohrkanüle in Blutadern wie zum Beispiel die Drosselvene ermöglicht wird.

## Revendications

1. Aiguille (4) comprenant des moyens de guidage est **caractérisée par** un manchon (1) localisé de manière coaxiale par rapport à l'aiguille (4) et à partir de ce manchon se projette une section collatérale (2) avec un passage tubulaire (3) prévu pour guider précisément une grande aiguille forée jusqu'à l'extrémité de l'aiguille avec des moyens de guidage (4), ledit passage tubulaire (3) étant orienté de manière à ce que l'axe de la grande aiguille forée coupe l'axe de l'aiguille avec des moyens de guidage (4), permettant par ce moyen une insertion sûre et précise de la grande aiguille forée jusqu'à l'intérieur de vaisseaux sanguins, telle que la veine jugulaire interne.
